(19) Europäisches Patentamt
European Patent Office
Office européen des brevets

(11) Publication number: **0 175 291 B1**

(12) **EUROPEAN PATENT SPECIFICATION**

(45) Date of publication of patent specification: **15.01.92**

(51) Int. Cl.⁵: **C12P 13/00**, C07C 323/26, C07C 317/06

(21) Application number: **85111558.4**

(22) Date of filing: **12.09.85**

(54) **Novel alpha-D-mannosidase inhibitor.**

(30) Priority: **21.09.84 JP 196761/84**

(43) Date of publication of application:
**26.03.86 Bulletin 86/13**

(45) Publication of the grant of the patent:
**15.01.92 Bulletin 92/03**

(84) Designated Contracting States:
**BE CH DE FR GB IT LI NL SE**

(56) References cited:

**CHEMICAL ABSTRACTS, vol. 67, no. 19, 6th November 1967, page 8251, abstract no. 87803u, Columbus, Ohio, US; M. URAMOTO et al.: "Mannosyl glucosaminide, a new antibiotic", & J. ANTIBIOT. (TOKYO), SER. A 20(4), 236-7**

(73) Proprietor: **MICROBIAL CHEMISTRY RESEARCH FOUNDATION**
**14-23, Kamiosaki 3 Chome Shinagawa-ku**
**Tokyo 141(JP)**

(72) Inventor: **Umezawa, Hamao, Prof.**
**23, Toyotama-kita 4-chome Nerima-ku**
**Tokyo(JP)**
Inventor: **Takeuchi, Tomio**
**1-11, Higashigotanda 5-chome Shinagawa-ku**
**Tokyo(JP)**
Inventor: **Aoyagi, Takaaki**
**3-6, Honkugenuma 3-chome**
**Fujisawa-city Kanagawa Prefecture(JP)**
Inventor: **Hamada, Masa**
**26-405, Naito-cho 1-chome Shinjuku-ku**
**Tokyo(JP)**
Inventor: **Kojiri, Katsuhisa**
**25-12, Senju 4-chome Adachi-ku**
**Tokyo(JP)**
Inventor: **Morishima, Hajime**
**1-32-301, Nakameguro 1-chome Meguro-ku**
**Tokyo(JP)**

(74) Representative: **Wächtershäuser, Günter, Dr.**
**Tal 29**
**W-8000 München 2(DE)**

EP 0 175 291 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Field of the Invention

In recent years, it has become clear that complex saccharides present on the surfaces of cells play essential roles in the important vital phenomena the cell surfaces take part in. Various phenomena such as immunological phenomena, inflammation, cell fusion, complement fixation, transformation into cancer cells, metastasis of cancer cells, and viral infection, are being recognized as problems with such complex saccharides on the cell surfaces, and extensive studies are proceeding on this topic. -D-mannose is one of the saccharides present on the cell surfaces, and an inhibitor of α-D-mannosidase, an enzyme hydrolyzing the saccharidal portion including α-D-mannosidase, is very useful in the fiel of pharmaceuticals.

### Description of the Prior Art

Swainsonine isolated from a plant of Leguminosae has been known as an inhibitor of α-D-mannosidase (Australian J. Chem., Vol. 32, 2257-2264, 1979). This substance has recently been isolated from Metarhizium anisoplie as well, and has been found to possess an imunomodulator action (Japanese Laid-Open Patent Publication 73519/84).

### Problem the Invention is to Solve

As mentioned above, Swainsonine inhibits α-D-mannosidase of the leguminous plant origin. The present inventors speculated that an inhibitor of α-D-mannosidase derived from a rat's epididymis might be more useful in the pharmaceutical field than an inhibitor of the enzyme of the plant origin. Based on this speculation, they made search for any inhibitors in the metabolites of microorganisms, and found that a species of actinomycetes produced an inhibitor of α-D-mannosidase originating from a rat's epididymis.

### Measure to Solve the Problem

The present inventors searched for inhibitors of α-D-mannosidase in the cultured broths of microorganisms. Their search led to the discovery that a strain of actinomycetes, ME3-AG3, which the Institute of Microbial Chemistry isolated in July 1973 from a soil sample collected in Kiso, Nagano Prefecture, Japan, produced an α-D-mannosidase inhibitor, mannostatin, having a novel structure.
The microbiological characteristics of the strain ME3-AG3 will be described below.

### Microbiological Characteristics of ME3-AG3

1. Morphology

Microscopically, substrate mycelia were branched and extended aerial hyphae having whirls. On the aerial hyphae, no spirals were observed. Chains of mature spores included those having more than 10 spores. The spores ranged in size from about 0.4 to 0.7 x 0.8 to 1.1 microns, and their surfaces were smooth.

2. Culture characteristics on various media

In the following disclosure, the standards indicated in square brackets [ ] to describe colors comply with the Color Harmony Manual of the container corporation of America.

(1) On sucrose-nitrate agar medium (cultured at 27° C):

Growth was colorless to pale yellow, and white aerial mycelium developed on the growth. No soluble pigment was produced.

(2) On glucose-asparagine agar medium (cultured at 27° C):

Growth was pale yellow [2ic, Honey Gold] to yellowish brown [3ng, Yelow Maple], and white aerial mycelium developed slightly on the growth. Dull yellow [2na, Brite Yellow to 3pc, Amber] soluble pigment was produced.

(3) On glycerol-asparagine agar medium (ISP-5, cultured at 27° C):

Growth was pale yellowish brown [3ne, Topaz] to yellowish brown [3ng, Yelow Maple], and white to yellowish gray aerial mycelium developed on the growth. Dull yellow to yellowish brown soluble pigment

was produced.

(4) On starch-inorganic salts agar medium (ISP-4, cultured at 27° C):

Growth was colorless to pale yellow, and white aerial mycelium developed on the growth. No soluble pigment was produced.

(5) On tyrosine agar medium (ISP-7, cultured at 27° C):

Growth was pale yellow (2ne, Mustard Gold] to yellowish brown [3ng, Yellow Maple], and white to yellowish gray or pale yellowish orange aerial mycelium developed on the growth. Slightly yellowish brown soluble pigment was produced.

(6) On nutrient agar medium (cultured at 27° C):

Growth was pale yellow to pale yellowish brown [31e, Cinnamon], and no aerial mycelium developed or slightly white to yellowish gray aerial mycelium developed. No soluble pigment was produced.

(7) On yeast extract-malt extract agar medium (ISP-2, cultured at 27° C):

Growth was pale brown [2pg, Mustard Gold] to yellowish brown [2pi, Mustard Brown], and white to yellowish gray [3ba, Shell Tint to 1ba, Yellow Tint] aerial mycelium developed. Slightly brownish soluble pigment was produced.

(8) On oatmeal agar medium (ISP-3, cultured at 27° C):

Growth was colorless to pale yellow, and no aerial mycelium developed on the growth. No soluble pigment was produced.

(9) On glycerol-nitrate agar medium (cultured at 27° C):

Growth was dull yellow to yellowish brown [3pi, Golden Brown] to dark brown [4pn, Dk Brown], and white to yellowish gray [3ba, Shell Tint] aerial mycelium slightly developed. Yellowish brown soluble pigment was produced.

(10) On starch agar medium (cultured at 27° C):

Growth was colorless, and no aerial mycelium developed or white aerial mycelium slightly developed. No soluble pigment was produced.

(11) On calcium malate agar medium (cultured at 27° C):

Growth was colorless to pale yellow, and no aerial mycelium developed or white aerial mycelium slightly developed. No soluble pigment was produced.

(12) On cellulose medium (cultured at 27° C):

Growth was colorless, and no aerial mycelium developed. No soluble pigment was produced.

(13) On gelatin stab culture medium:

On simple gelatin medium (cultured at 20° C), growth was colorless to pale yellow to yellowish brown; no aerial mycelium developed; and slightly brown soluble pigment was produced. On glucose-peptone-gelatin medium (cultured at 27° C), growth was colorless to pale yellow; no aerial mycelium developed; and no soluble pigment was produced.

(14) On skimmed milk medium (cultured at 37° C):

Growth was colorless to pale yellow or pale yellowish brown, and no aerial mycelium developed. No soluble pigment was produced.

3. Physiological properties

(1) Growth temperatures:

Tests for growth were carried out at temperatures of 20, 24, 27, 30, 37 and 50° C on maltose-yeast extract agar medium (1.0% maltose, 0.4% yeast extract (Oriental), 3.5% agar string, pH 6.0). Growth occurred at all these temperatures, except 50° C. The optimum temperature was considered to be about 37° C.

(2) Liquefaction of gelatin (glucose-peptone-gelatin:

cultured at 20° C):

Liquefaction did not take place on the simple gelatin medium. Slight liquefaction began after about 4 days of culture, but the liquefactive strength was very weak.

(3) Hydrolysis of starch (tested on starch-inorganic salts agar medium, and starch agar medium each cultured at 27° C):

Hydrolysis was not observed after 21 days of culture.

(4) coagulation and peptonization of skimmed mild (cultured at 37° C):

Coagulation was completed at about 3 days of culture, and peptonization immediately began. The strength was intermediate to high.

(5) Production of melanoid pigment (tested on tryptone-yeast extract-broth medium (ISP-1), peptone-yeast extract-iron agar medium (ISP-6), tyrosine agar medium (ISP-7,), each cultured at 27° C):

No melanid pigment was produced on any of the media.

(6) Utilization of carbon sources (tested on Pridham-Gottlieb agar medium (ISP-9), cultured at 27° C):

Glucose was utilized. Raffinose was evaluated to be probably utilized. L-arabinose, D-xylose, D-fructose, sucrose, inositol, rhamnose of D-mannitol was not utilized.

(7) Dissolution of calcium malate (tested on calcium malate agar medium, cultured at 27° C):

Calcium malate was occasionally dissolved around the growth after about 21 days of cultivation. The dissolving strength was very weak.

(8) Reduction of nitrate (tested on 0.1% potassium nitrate-containing peptone water medium (ISP-8), cultured at 27° C):

Reduction was negative.

The characteristics of the strain ME3-AG3 described above can be summarized as follows:

Morphologically, no sporangia are seen, and whirls are formed on aerial hyphae, but no spirals are formed. The surfaces of spores are smooth.

Growths on various media are pale yellow to yellowish brown, and no aerial mycelia develop, or white to yellowish gray aerial mycelia develop on th growths. Dull yellow to yellowish brown soluble pigments may be produced, but in most cases, no soluble pigments are produced. Melanoid pigment production is negative on any of the media tested. Hydrolysis of starch does not occur. Proteolytic activity is weak for liquefaction of gelatin, but intermediate to strong for the coagulation and peptonization of skimmed milk.

Furthermore, the 2,6-diaminopimelic acid contained in this strain is of the LL-type. This fact considered in combination with the above-mentioned properties shows this strain to belong to the genus Streptoverticillium.

Search on known species based on these properties has shown the strain ME3-AG3 to be closely related to Streptomyces verticillus, the strain producing bleomycin and phleomycin (Japanese Patent Aplication No. 2598//59; the Journal of Antibiotics, Ser. A, Vol. 12, page 111, 1959; The Journal of Antiboitics, Ser. A, Vol. 19, page 200, 1966), and Streptomyces verticillus var. quintum, the strain producing the physiologically active substance siastatin and bleomycin (Japanese Patent Applicaiton No. 46714/80; The Journal of Antibiotics, Vol. 27, page 963).

The strain ME3-AG3 is considered to be more closely related to Streptomyces verticillus var. quintum in the following respects: Production of phleomycin in addition to mannostatin; characteristic yellowish brown growths; intermediate to strong acitvity of coagulating and peptonizing milk; no reduction of nitrate; and no hydrolysis of starch.

Therefore, Streptomyces verticillus var. quintum MB695-A4 No. IMC S-0666 and the strain ME3-AG3 were compared. The results will be summarized below.

| | ME3-AG3 | Streptomyces verticillus var. quintum IMC S-0666 (MB695-A4) |
|---|---|---|
| Formation of whirls | + | + |
| Formation of spirals | - | - |
| Surface of spore | Smooth | Smooth |
| Color of aerial mycelim | White to yellowish gray | White to yellowish |
| Color of growth | Pale yellow to yellowish brown | Pale yellow to dark yellow |
| Soluble pigment | - to yellowish brown | - |
| Decomposition of cellulose | - | + |
| Production of melanoid pigment | | |
| ISP-1 | - | - |
| ISP-6 | - | - |
| ISP-7 | - | - |
| Hydrolysis of starch | - | - |
| Coagulation of milk | + | + |
| Peptonization of milk | + | + |
| Liquefaction of gelatin | | |
| Simple gelatin | - | - |
| Glucose-peptone-gelatin | + weak | + weak |
| Reduction of nitrate | - | - |
| Utilization of carbon sources | | |
| Glucose | + | + |
| Arabinose | - | - |
| Xylose | - | - |
| Fructose | - | - |
| Sucrose | - | - |
| Inositol | - | - |
| Rhamnose | - | - |
| Raffinose | ± | ± |
| Mannitol | - | - |

±: Probably utilized.

The strain ME3-AG3 was deposited on October 14, 1983 with the Fermentation Research Institute, Agency of Industrial Science and Technology, Ministry of International Trade and Industry of Japan under the accession number FERM-P 7299.

The compounds of this invention are obtained by inoculating media containing nutritional sources with spores or mycelia of the strain ME3-AG3, the strain of Streptomyces that produces those compounds, and then culturing the inocula aerobically. The nutritional sources may be any sources which are known to be utilized by actinomycetes. Examples are carbon sources such as carbohydrates, e.g. glycerol, glucose, lactose, sucrose, starch, maltose and molasses or fats and oils (animal fats, and plant oils such as soybean

oil, cotton seed oil or peanut oil); commercially available nitrogen sources such as peptone, meat extract, corn steep liquor, cotton seed meal, peanut meal, soybean meal, yeast extract, N-Z amine, casein, sodium nitrate, ammonium nitrate and ammonium sulfate; and inorganic salts such as sodium chloride, phosphates, calcium carbonate, and magnesium sulfate. Trace amounts of metal salts may be further added if desired. They may be any materials which can be utilized by the mannostatin-producing strain and which are useful for the production of mannostatin. All of materials for culture of known actinomycetes can be utilized. Fats and fatty acids are used in the biosynthesis of mannostatin, and can be utilized not only as anti-foaming agents, but as carbon sources for the production of mannostatin.

To mass-produce mannostatin, liquid culture is preferred. The cultivation temperature may be any temperature at which the mannostatin-producing microorganism is grown to produce mannostatin. The preferred temperature is 25 to 35 °C. The cultivation is continued ususally until this substance is produced and accumulated in sufficient amounts in the culture broth. For instance, the cultivation is carried out as folows. A culture medium (2% glycerol, 1.5% polypeptone, 0.3% yeast extract and 0.3% $CaCO_3$, pH 7.2) that has been sterilized is inoculated with spores and mycelia from a slant culture of the strain ME3-AG3. The inoculated medium is shake-cultured aerobically at 28 °C. The accumulation of mannostatin in the medium is recognized usually after 3 to 4 days of cultivation.

Mannostatin can be produced statifactorily by jar fermentation as in the case of shake culture. For example, 130 liters of a culture medium was placed in a 200-liter jar fermentor, sterilized, and inoculated with the mannostatin- producing organism. Then, the inoculated medium was stirred at 200 rpm under aeration with 130 liters of sterile air per minute. The produciton of mannostatin peaked in 72 hours.

The compounds of this invention are present mainly in the cultured broth of the mannostatin-producing organism. These comounds are separated from the cultured broth by measures which are usually used to recover metabolites produced by microorganisms from the cultures of the microorganisms.

Since the compounds of this invention are water-soluble basic substances, they can be isolated, for example, by the following methods of separation and purification. The filtrate of the culture broth is poured upon a column packed with 1% volume of chromatographic activated carbon (Wako Jnyaku) for decolorization. The activated carbon is washed with 1.5- to 2-fold volume of 0.2M formic acid, and the washings are combined with the decolorized filtrate. The combined liquid is layered over Dowex® 50W x 2 ($H^+$ type), and eluted with 0.5N hydrochloric acid. The eluate is adjusted to a pH of 5.0 with sodium hydroxide, and then poured upon a column packed with 1% (g/V) of chromatographic activated carbon for adsorption. The column is eluted wiht 0.2M formic acid, and the active fractions are combined. The combined fraction is adsorbed to Dowex® 50W x 8 ($H^+$ type), and eluted with 0.3N hydrochloric acid, thereby dividing the active fractions into two types. The active fractions to appear first are composed of mannostatin S-oxide, and the active fractions to come next comprise mannostatin. The mannostatin S-oxide fractions are adjusted to pH 10 with a solution of sodium carbonate, and poured upon a column packed with chromatographic activated carbon for adsorption. After washing with water, the column is eluted with 0.2M formic acid. The acitve fractions are combined, adjusted to pH 7.0 with Dowex® 1 x 2 ($OH^-$ type), and filtered. The filtrate is layered over a column of Dowex® 1 x2 that has been equilibrated with 0.05M carbonate buffer (pH 10.0), and is then eluted with the same buffer. The active fractions are combined, and poured upon a column of chromatographic acitivated carbon for adsorption. After washing with water, the column is eluted with 0.2N hydrochloric acid. The active fractions are combined, adjusted to ph 5.0 with Dowex® 1 x2 ($OH^-$ type), and filtered. The filtrate is evaporated to dryness to obtain slightly yellow solids.

The resulting mannostatin S-oxide shows a single spot on high-voltage paper electophoresis. The mannostatin fractions are also subjected to the same procedure, whereby slightly yellow, solid mannostatin can be obtained which shows a single spot on high-voltage paper electophoresis.

The physicochemical properties of mannostatin and its S-oxide will be described below.

These compounds are stable under neutral, acidic and basic conditions. When aqueous solutions of those compounds are allowed to stand for 48 hours at pHs of 2.0, 7.0 and 9.0, for example, the -D-mannosidase inhibitory activity does not lower. The two compounds are highly soluble in water, and sparingly soluble in acetone, chloroform and benzene.

Both substances produce positive ninhydrin, Ehrlich and potassium permanganate reactions, and give positive reactions when heated with sodium formate and applied to a lead acetate paper. They give negative results in 2,4-dinitrophenylhydrazine test, Rydon-Smith reaction, Fehling test, and Sakaguchi reaction.

When both substances were thin-layer chromatographed on silica gel (E. Merck, West Germany) and developed with n-butanol-acetic acid-water (3:1:1), the Rf value was 0.23 for mannostatin S-oxide and 0.43 for mannostatin. On high-voltage paper electrophoresis (600 V, 30 minutes) using formic acid-acetic acid-water (25:75:900, pH 1.8), both substances showed an Rm value of 1.07 with that of alanine being 1.

Both substances are hygroscopic, but can be obtained as crystals when acetylated. Tetraacetate of mannostatin S-oxide is obtained by suspending mannostatin S-oxide in pyridine, and reacting the suspension at 40°C for 3 hours with the addition of acetic anhydride. Crystallization from a mixture of ethyl acetate and n-hexane gives white crystals. The tetracetate had a melting point of 146°C (decomp.) and showed the following values on elemental analysis: C: 46.41%, H: 5.80%, N: 3.80%, S: 8.87%.

The infrared absorption spectrum of the tetraacetate of mannostatin S-oxide showed absorptions at 3260, 3040, 2920, 1738, 1645, 1540, 1422, 1400, 1375, 1295, 1250, 1218, 1125, 1100, 1065, 1015, 970, 940, 918, 900, 875, 830, 795, 762 and 675 $cm^{-1}$.

Tetraacetate of mannostatin is obtained by suspending mannostatin in pyridine, and heating the suspension at 40°C for 1 hour with the addition of acetic anhydride with stirring. Crystallization from benzene and n-hexane gives white needle crystals. The melting point of mannostatin tetraacetate was 121°C (decomp.) and its elemental analysis showed the following values: C: 48.86%, H: 5.95%, N: 3.99%, S: 9.28%.

The infrared absorption spectrum of the mannostatin tetraacetate showed absorptions at 3290, 3040, 2970, 1740, 1725, 1690, 1635, 1530, 1430, 1370, 1355, 1300, 1280, 1260, 1235, 1100, 1080, 1040, 1010, 975, 955, 950, 935, 925, 905, 885, 865, 815, 730, and 695 $cm^{-1}$.

Mannostatin S-oxide can be converted into mannostatin, as will be easily anticipated from its structure, by adding thioglycolic acid to an aqueous solution of mannostatin S-oxide, and reducing the mixture at 50°C for 30 hours.

Structure determination by the present inventors has shown the compounds of this invention to be cyclopentanols having the following novel structures:

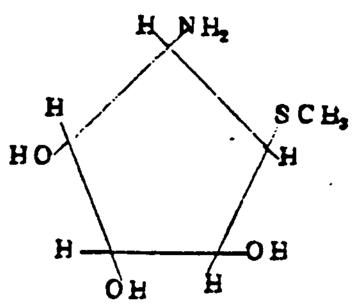

Mannostatin

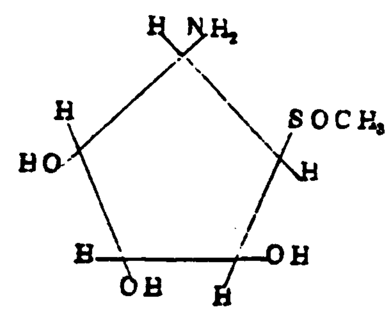

Mannostatin S-oxide

Action

Assay of the compounds according to this invention is performed by measuring their α-D-mannosidase inhibitory activity. The activity of α-D-mannosidase is determined by colorimetrically measuring the amount of p-nitrophenol that has been liberated when p-nitrophenyl-α-D-mannopyranoside (Koch-Light Laboratories, England) as a substrate is hydrolyzed with α-D-mannosidase of the rat epididymis origin (a fraction obtained by precipitation with 20 - 80% ammonium sulfate at the Institute of Microbial Chemistry). Details of said procedure are as follows: p-Nitro-phenyl-α-D-mannopyranoside is dissolved in 50% methanol to make a 0.04M solution. To 0.05 ml of the 0.04M substrate solution are added 0.25 ml of a 0.1M acetate buffer (pH 4.5) and 0.15 ml of water or an aqueous solution containing the test compound. The mixture is heated at 37°C for 3 minutes, and 0.05 ml of -D-mannosidase is added. The α-D-mannosidase has been obtained as an enzyme solution by dissolving an ammonium sulfate-precipitated fraction, which has been obtained from the epoididymides of 20 rats, in 45 ml of 0.02M zinc acetate, and diluting the solution with 0.02M zinc acetate to 20 to 30 times the original volume. The aforementioned mixture containing α-D-mannosidase is reacted at 37°C. After 20 minutes of the reaction, 2.0 ml of 0.4M glycine-sodium hydroxide buffer (pH 10.5) is added to terminate the reaction. The reaction mixture is measured for the absorbance (A) at 400 nm. Separately, the control mixture comprising only water and being free from the aforesaid test compound solution is measured for the absorbance termed (b). The α-D-mannosidase inhibition rate (%) is calculated from the equation (b - a)/b x 100.

According to this procedure, mannostatin and its S-oxide in an amount of 0.005 mcg each produced 50% inhibition ($IC_{50}$). The $IC_{50}$ was described as the concentration in 0.45 ml of the reaction mixture.

## Examples

The following Examples concern the recovery of mannostatin and its S-oxide. This invention, however, is in no way restricted to these Examples, because it may be modified based on the findings in this specification which show the properties, and methods of production, extraction and purification, of those compounds elucidated by the present inventors.

### Example 1

A culture medium was prepared according to the following recipe:

| | |
|---|---|
| Glycerol | 2.0% |
| Polypeptone (Daigo Eiyo Kagaku) | 1.5% |
| Yeast extract | 0.3% |
| $CaCO_3$ | 0.3% |

125 ml of the above medium was charged into each of 500 ml Erlenmeyer flasks for cultivation, and sterilized for 20 minutes at 120° C. The media in the flasks were inoculated with a loopful of spores and mycelia from a slant culture of the actinomycete strain ME3-AG3 having the ability to produce a substance showing an -D-mannosidase inhibitory acitivty. The inoculated media were pre-cultured for 4 days at 27° C at 180 rpm to prepare a seed culture. The pH of the media was 7.2 before inoculation, 7.2 after 1 day, 5.0 after 2 days, 4.68 after 3 days, and 4.73 after 4 days of cultivaiton. 10 ml of the seed culture was inoculated to each of 5-liter Erlenmeyer flasks each containing 1.25 liters of the aforementioned medium that had been sterilized. The inoculated flasks were cultured for 89 hours at 27° C at 180 rpm. The cultured broth was filtered to separate the bacterial cells and obtain 25.2 liters of a clear filtrate. The filtrate had a pH of 6.2 and produced 50% inhibition of α-D-mannosidase in an amount of 0.24 µl. The filtrate was poured upon a column packed with 1.5 liters of chromatographic activated carbon (Wako Junyaku), and the column was washed with 3 liters of 0.2M formic acid. The washings were combined with said filtrate that had been passed through the column. The combined fluid was adsorbed to a column packed with 1.87 liters of Dowex® 50W X2 ($H^+$ type). After washing with 8 liters of water, the column was eluted with 0.5N hydrochloric acid. The active fractions were combined to obtain 9 liters of a solution. The $IC_{50}$ of this solution was 0.1 µl.

### Example 2

A solution containing an α-D-mannosidase inhibitor obtained in the same way s in Example 1 was adjusted to pH 7.8 with 10N sodium hydroxide. 9.35 liters of this solution was adsorbed to a column packed with 400 ml of chromatographic activated carbon. After washing with 3.5 liters of water, the column was eluted with 0.2M formic acid. The active fractions were combined to obtain 710 ml of a solution. The $IC_{50}$ of this solution was 0.009 µl.

### Example 3

an α-D-mannosidase inhibitor-containig solution obtained in the same way as in Example 2 was layered over a column (3 x 30 cm) packed with Dowex® 50W X8 ($H^+$ type). The overlaid material eluted with 0.3N hydrochloric acid to obtain fractions weighing 18 g each. The substances having α-D-mannosidase inhibitor acitvity were detected in Fractions Nos. 138 to 176, and Fractions Nos. 210 to 262. The former fractions termed Fractions A (mannostatin S-oxide fractions) amounted to 530 ml. The latter fractions termed Fractions B (mannostatin fractions) amounted to 760 ml. The $IC_{50}$ of Fractions A was 0.012 µl, while that of Fractions B was 0.028 µl. These Fractions A and B were adjusted to pH 5.5 with Dowex® 1 X2 ($OH^-$ type) and concentrated to dryness to obtain 2.37 g of the active substance from Fractions A. This substance showed $IC_{50}$ at 0.05 µg. From Fractions B was obtained 296 mg of the active substance showing $IC_{50}$ at 0.013 µg.

### Example 4

10.2 g of Fractions A obtained in the same way as in Example 3 was dissolved in 500 ml of water, and

adjusted to pH 10.0 with 0.5M sodium carbonate. The solution was poured upon a column packed with 100 ml of chromatographic activated carbon (Wako Junyaku), and the column was washed with 1,500 ml of distilled water, followed by elution with 0.2M formic acid. The active fractions were combined to obtain a 330 ml solution. This solution showed $IC_{50}$ at 0.0028 μl. The solution containing the active principle was adjusted to pH 7.0 with Dowex® 1 X2 ($OH^-$ type), and then concentrated. The concentrate was layered over a column (2.1 x 62 cm) of Dowex® 1 X8 equilibrated with 0. 05M carbonate buffer (pH 10.0). The overlaid material was eluted with 0.05M carbonate buffer (pH 10.0), and the active fractions were combined to obtain a 200 ml active fraction. This fraction showed $IC_{50}$ at 0.00196 μl.

Example 4

10.2 g of Fractions A obtained in the same way as in Exampel 3 was dissolved in 500 ml of water, and adjusted to pH 10.0 with 0.5M sodium carbonate. The solution was poured upon a column packed with 100 ml of chromatographic acitivated carbon (Wako Junyaku), and ;the column was washed with 1,500 ml of distilled water, followed by elution with 0.2M formic acid. The active fractions were combined to obtain a 330 ml solution. This solution showed $IC_{50}$ at 0.0028 ul. The solution containing the active principle was adjusted to pH 7.0 with Dowex® 1 X2 ($OH^-$ type), and then concentrated. The concentrate was layered over a column (2.1 x 62 cm) of Dowex® 1 X8 equilibrated with 0.05M carbonate buffer (pH 10.0). The overlaid material was eluted with 0.05M carbonate buffer (pH 10.0), and the acitve fractions were combined to obtain a 200 ml active fraction. This fraction showed $IC_{50}$ at 0.00196 μl.

Example 5

The active fraction from Fractions A obtained in Example 4 was layered over a column packed with 100 ml of chromatographic activated carbon, and washed with 800 ml of distilled water. The overlaid material was eluted with 0.2N hydrochloric acid, and the active fractions were combined. The combined fraction was adjusted to pH 5.0 with Dowex® 1 X2 ($OH^-$ type), and concentrated to dryness to obtain 473 mg of mannostatin S-oxide. Its $IC_{50}$ was 0.005 μg.

Example 6

122 mg of Fractions B obtained in the same way as in Exampel 3 was dissolved in 3 ml of 0.05M carbonate buffer (pH 10.0). The solution was layered over a column (2.1 x 64 cm) of Dowex® 1 X8 equilibrated with 0.05M carbonate buffer (pH 10.0), and eluted with the same buffer. The active fractions were combined to obtain a 260 ml active fraction. The active fractions were combined to obtain a 260 ml active fraction. The $IC_{50}$ of this active fraction was 0.019 ml.

Example 7

An active fraction from Fractions B that had been obtained in the same way as in Example 6 was layered over a column of 50 ml of chromatographic acitivated carbon. After washing with 1,000 ml of distilled water, the overlaid material was eluted with 0.2N hydrochloric acid. The active fractions were combined, and adjusted to pH 5.0 with Dowex® 1 X2 ($OH^-$ type). Then, the combined fraction was concentrated to dryness to obtain 43 mg of mannostatin. Its $IC_{50}$ was 0.0051 μg.

Exampel 8

130 liters of the same culture medium as in Example 1 was charged into a 200-liter stainless steel fermentor, and sterilized for 30 minutes at 115 °C. Then, 1 liter of ageed culture of the strain ME3-AG3 resulting from 48 hours of shake culture was inoculated to the medium, and the inoculated medium was cultured for 72 hours at 28 °C under aeration with 130 liters of air with stirring at 200 rpm. The fermentation broth was filtered to separate the bacterial cells and obtain 115 liters of a clear filtrate (pH 5.7, $IC_{50}$ = 0.32 μl). The filtrate was poured upon a column packed with 7.5 liters of chromatographic activated carbon (Wako Junyaku). The column was washed with 0.2M formic acid, and 15 liters of the washings were combined with said filtrate that had passed through the column. The combined fluid was poured upon a column of 10 liters of Amberlite® IR 120 ($H^+$ type), and the column was washed with water. The column was then eluted with 0.5N hydrochloric acid to obtain a 50 liter eluate. The $IC_{50}$ of this eluate was 0.23 μl. The eluate was adjusted to pH 8 with 30% sodium hydroxide, and poured upon a 2 liter column of

chromatographic activated carbon. After washing with 15 liters of water, the column was eluted with 0.2M formic acid to obtain a 9 liter eluate. The $IC_{50}$ of this eluate was 0.05 µl. The eluate was poured upon a 200 ml column of Dowex® 50W X8 ($H^+$ type). After washing with 1 liter of water, athe column was eluted with 0.3N hydrochloric acid to obtain 980 ml of Active Fractions A to appear first, and 470 ml of Active Fractions B to come next. The $IC_{50}$ was 0.0083 µl for Active Fractions A and 0.036 µl for Active Fractions B. The Active Fractions A and B were adusted to pH 5.5 with Dowex® 1 X2 ($OH^-$ type), and subjected to the following procedure:

Active Fraction A were concentrated with to 200 ml, and the concentrate was adjusted to pH 10.0 with 0.5M sodium carbonate. The concentrate was then poured upon a column packed with 200 ml of chromatographic activated carbon, and washed with 2000 ml of water, followed by elution with 0.2M formic acid. The active fractions were combined to obtain a 500 ml active fraction having an $IC^{50}$ of 0.0051 µl. This active fraction was adjusted to pH 7.0 with Dowex® 1 X2 ($OH^-$ type), concentrated, and layered over a column (2.9 x 91 cm) of Dowex® 1 X8 equilibrated with 0.05M carbonate buffer (pH 10.0). The overlaid material was eluted with the same buffer, and the active fractions were combined. The combined fluid was placed on a 200 ml column of chromatographic activated carbon. After washing with 2000 ml of water, the overlaid material was eluted with 0.2N hydrochloric acid. The active fractions were combined, adjusted to pH 5.0 with Dowex® 1 X2 ($OH^-$ type), and concentrated to dryness ot obtain 355 mg of mannostatin S-oxide. The $IC_{50}$ of this solid was 0.005 µg.

Active Fractions B were concentrated to dryness, and dissolved in 10 ml of 0.05M carbonate buffer (pH 10.0). The solution was layered over a column (2.1 x 64 cm) of Dowex® 1 X8 equilibrated with 0.05M carbonate buffer (pH 10.0), and then eluted with the same buffer. The active fractions were combined, supreimposed on a 50 ml column of chromatographic activated carbon, and washed with 1000 ml of water, followed by elution with 0.2N hydrochloric acid. The active fractions were combined, adjusted to pH 5.0 with Dowex® 1 X2 ($OH^-$ type), and concentrated to dryness to obtain 355 mg of mannostatin S-oxide. The $IC_{50}$ of this solid was 0.005 µg.

Active Fractions B were concentrated to dryness, and dissolved in 10 ml of 0.05M carbonate buffer (pH 10.0). The solution was layered over a column (2.1 x 64 cm) of Dowex® 1 X8 equilibrated with 0.05M carbonate buffer (pH 10.0), and then eluted with the same buffer. The active fractions were conbined, supreimposed on a 50 ml column of chromatographic activated carbon, and washed with 1000 ml of water, followed by elution with 0.2N hydrochloric acid. The active fractions were combined, adjusted to pH 5.0 with Dowex® 1 X2 ($OH^-$ type), and concentrated to dryness to obtain 40 mg of mannostatin. The $IC_{50}$ of this solid was 0.0051 µg.

Efficacy of this invention

It has been made clear that mannostatin and its S-oxide have novel cyclopentanol structures containing an amino group, methylthio group or methylsulfinyl group which are not easily predictable from mannose derivatives, the substrate. It has been also clarified that these substances stronghly inhibit α-D-mannosidase of the animal origin (rat epididymides).

Concanavalin A has been known to induce the blastogenesis of cells when adhering to the α-D-mannosidase portion on the surfaces of the cells. Therefore, α-D-mannose derivatives or inhibitors of α-D-mannosidase may suppress this blastogenesis, thereby impeding trasformation into cancer cells and metastases of cancer and acting on the immune system. Actually, swainsonine, the inhibitor of α-D-mannosidase from leguminous plants, is known to have an immunomodulator action.

As noted above, mannostatin and its S-oxide strongly inhibit α-D-mannosidase of the animal origin. Thus, they are more likely to inhibit transformation into cancer cells and cancer metastasis, and to show effects on the immune system. They are very useful in the pharmaceutical field.

## Claims

**1.** α-D-mannosidase inhibitor, mannostatin, or its S-oxide, expressed by the following formula (1)

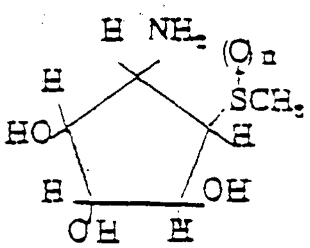

(1)

wherein n denotes 0 or 1.

**2.** A process for producing α-D-mannosidase inhibitor, mannostatin, or its S-oxide, expressed by the following formula (1)

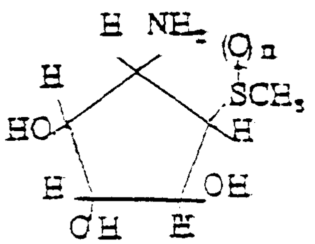

(1)

wherein n denotes 0 or 1,

which comprises culturing an actinomycete of the genus Streptomyces (FERM-P No. 7299 FERM BP-839), said actinomycete having the ability to produce said compound of the formula (1).

## Revendications

**1.** Inhibiteur de l'α-D-mannosidase, à savoir la mannostatine, ou son S-oxyde, représenté par la formule (1) suivante :

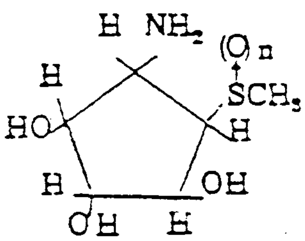

(1)

dans laquelle n désigne 0 ou 1.

**2.** Procédé pour la production d'un inhibiteur de l'α-D-mannosidase, à savoir la mannostatine, ou son S-oxyde, représenté par la formule (1) suivante :

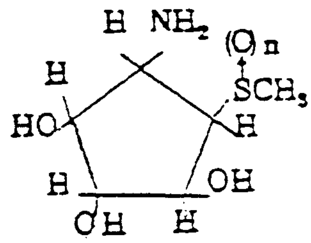

(1)

dans laquelle n désigne 0 ou 1,
qui comprend la culture d'un actinomycète du genre Streptomyces (FERM-P N° 7299 - FERM BP-839), ledit actinomycète possédant l'aptitude à produire ledit composé de la formule (1).

## Patentansprüche

1. α-D-Mannosidaseinhibitor, Mannostatin, oder dessen S-Oxid, dargestellt durch die folgende Formel (1)

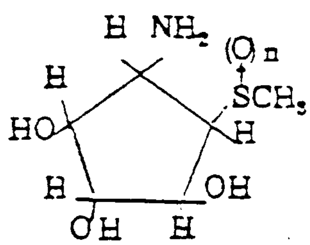

(1)

wobei n für 0 oder 1 steht.

2. Verfahren zur Herstellung von α-D-Mannosidaseinhibitor, Mannostatin, oder dessen S-Oxid, dargestellt durch die folgende Formel (1)

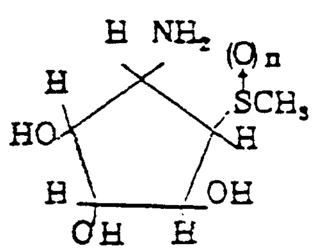

(1)

wobei n für 0 oder 1 steht, umfassend die Kultivierung eines Actinomyceten der Gattung Streptomyces (Ferm-P Nr.7299, Ferm BP-839), wobei der erwähnte Actinomycete die Fähigkeit hat, die erwähnte Verbindung der Formel (1) zu erzeugen.